(19)

Europäisches Patentamt
European Patent Office
Office européen des brevets

(11) **EP 4 439 575 A1**

(12) **EUROPEAN PATENT APPLICATION**

(43) Date of publication:
**02.10.2024 Bulletin 2024/40**

(21) Application number: **24167425.8**

(22) Date of filing: **28.03.2024**

(51) International Patent Classification (IPC):
**G16H 20/17** (2018.01)     **A61M 5/172** (2006.01)
**A61B 5/145** (2006.01)     **A61B 5/00** (2006.01)
**A61M 5/142** (2006.01)

(52) Cooperative Patent Classification (CPC):
**G16H 20/17; A61B 5/14532; A61B 5/4839;
A61B 5/7275; A61M 5/14244; A61M 5/1723**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **28.03.2023   US 202363492564 P**

(71) Applicant: **Insulet Corporation
Acton MA 01720 (US)**

(72) Inventors:
• **LEE, Joon Bok
  Acton, 01720 (US)**
• **D'ARCO, John
  Wilmington, 01887 (US)**
• **ZHENG, Yibin
  Hartland, 53029 (US)**
• **O'CONNOR, Jason
  Acton, 01720 (US)**

(74) Representative: **Peterreins Schley
Patent- und Rechtsanwälte PartG mbB
Hermann-Sack-Straße 3
80331 München (DE)**

(54) **METHODS TO INCORPORATE SUBJECTIVE USER GLUCOSE CONCENTRATION FEEDBACK IN AUTOMATED INSULIN DELIVERY SYSTEMS**

(57)     A drug delivery system including a memory storing programming code operable to control delivery of a drug, a user interface operable to accept an input indicating a perceived glucose state of a user, and a processor operable to execute the programming code. When executed, the programming code causes the processor to: receive a perceived state signal from the user interface indicating the perceived glucose state of the user, evaluate the perceived glucose state of the user indicated by the perceived state signal using a glucose measurement value corresponding in time to when the perceived state signal was received, determine an adjustment to a drug delivery algorithm, and utilize the adjustment in a determination of a drug delivery dosage to be administered to a user.

FIG. 3

EP 4 439 575 A1

**Description**

**TECHNICAL FIELD**

The disclosed embodiments generally relate to medication delivery. More particularly, the disclosed embodiments relate to techniques and processes that incorporate subjective user glucose concentration feedback in automated insulin delivery system and devices.

**BACKGROUND**

Glucose concentration measurements are used by automated insulin delivery (AID) systems to adjust subcutaneous deliveries provided to users. However, variations in life patterns and delays inherent to subcutaneous sensors can delay the response of AID systems to rapid changes in glucose concentrations. In some cases, this delayed response can expose users to increased risks of hyperglycemia and hypoglycemia.

Accordingly, there is a need for an improved technique to identify changes in user glucose concentrations such that AID or automated drug delivery (ADD) systems can adjust subcutaneous deliveries with reduced delay.

**SUMMARY**

The present disclosure is directed to a drug delivery system as defined in the enclosed claims. The drug delivery system includes a memory storing programming code operable to control delivery of a drug, a user interface operable to accept an input indicating a perceived glucose state of a user, and a processor operable to execute the programming code. When executed, the programming code causes the processor to: receive a perceived state signal from the user interface indicating the perceived glucose state of the user, evaluate the perceived glucose state of the user indicated by the perceived state signal using a glucose measurement value corresponding in time to when the perceived state signal was received, determine an adjustment to a drug delivery algorithm, and utilize the adjustment in a determination of a drug delivery dosage to be administered to a user.

Another aspect of the present disclosure is directed to a method for adjusting a drug delivery dosage based on a user's perceived glucose state. The method includes receiving a signal from the user interface indicating a perceived glucose state of the user, evaluating the perceived glucose state of the user indicated by the signal using a glucose measurement value corresponding in time to when the signal was received, determining an adjustment to a drug delivery algorithm based on the perceived glucose state, and utilizing the adjustment in a determination of a drug delivery dosage to be administered to a user.

Another aspect of the present disclosure is directed to a drug delivery system. The drug delivery system includes a memory storing programming code operable to control delivery of insulin according to a predetermined insulin dosage, a user interface operable to accept an input indicating a perceived glucose state of a user, and a processor operable to execute the programming code. When executed, the programming code causes the processor to: receive, at a first time from the user interface, a perceived state signal indicating a perceived state of a user's glucose, retrieve a glucose measurement value closest in time to the first time, evaluate how well the glucose measurement value corroborates the perceived state of the user's glucose indicated by the received perceived state signal, and modify delivery of an insulin dosage based on the evaluation.

**BRIEF DESCRIPTION OF THE DRAWINGS**

In the drawings, like reference characters generally refer to the same parts throughout the different views. In the following description, various embodiments of the present disclosure are described with reference to the following drawings, in which:

FIG. 1 illustrates a block diagram of a drug delivery system according to embodiments of the present disclosure;
FIG. 2A illustrates a block diagram of a drug delivery system according to embodiments of the present disclosure;
FIG. 2B illustrates a perspective view of an example of a drug delivery system of FIG. 2A according to embodiments of the present disclosure;
FIG. 3 illustrates a user interface of a drug delivery system according to embodiments of the present disclosure;
FIG. 4 illustrates a flow diagram of an example method for evaluating a user's perceived glucose state and assigning a trust index to the user based on the evaluation according to embodiments of the present disclosure;
FIG. 5A illustrates a flow diagram of an example method for determining a trust level associated with each button press performed by the user according to embodiments of the present disclosure;
FIG. 5B illustrates a series of example glucose measurements being evaluated using the method of FIG. 5A;

**FIG. 6** a flow diagram of an example method for determining a trust index associated with a user according to embodiments of the present disclosure; and

**FIG. 7** illustrates an example computing device suitable for implementing and using the disclosed embodiments.

The drawings are not necessarily to scale. The drawings are merely representations, not intended to portray specific parameters of the disclosure. The drawings are intended to depict exemplary embodiments of the disclosure, and therefore are not to be considered as limiting in scope. Furthermore, certain elements in some of the figures may be omitted, or illustrated not-to-scale, for illustrative clarity. Still furthermore, for clarity, some reference numbers may be omitted in certain drawings.

## DETAILED DESCRIPTION

Systems, devices, and methods in accordance with the present disclosure will now be described more fully hereinafter with reference to the accompanying drawings, where one or more embodiments are shown. The systems, devices, and methods may be embodied in many different forms and are not to be construed as being limited to the embodiments set forth herein. Instead, these embodiments are provided so the disclosure is thorough and complete, and fully conveys the scope of methods and devices to those skilled in the art. Each of the systems, devices, and methods disclosed herein provides one or more advantages over conventional systems, components, and methods.

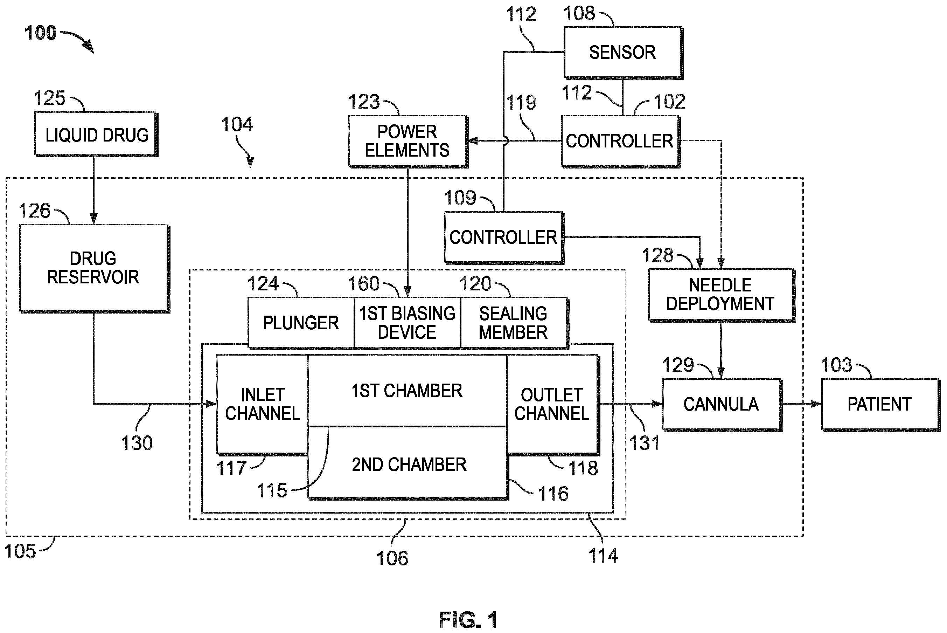

FIG. 1 illustrates a simplified block diagram of an example system 100. The system 100 may be a wearable or on-body drug delivery device and/or an analyte sensor attached to the skin of a patient 103. The system 100 may include a controller 102, a pump mechanism 104 (hereinafter "pump 104"), and a sensor 108. In some examples, the controller 102 is included in an external device (e.g., a cell phone or a personal diabetes management (PDM) device). The sensor 108 may be one or more of a glucose or other analyte monitor such as, for example, a continuous glucose monitor, a ketone sensor, a heart rate monitor, a blood oxygen sensor element, or the like, and may be incorporated into the wearable device. The sensor(s) 108 may, for example, be operable to measure glucose values of a user to generate a measured glucose level signal 112. The controller 102, the pump 104, and the sensor(s) 108 may be communicatively coupled to one another via a wired or wireless communication path. For example, each of the controller 102, the pump 104 and the sensor(s) 108 may be equipped with a wireless radio frequency transceiver operable to communicate via one or more communication protocols, such as Bluetooth®, or the like. As described in greater detail herein, the system 100 may also include a pump 104 which includes a drive mechanism 106 having at least one housing 114 defining a pump chamber 115, a channel chamber 116, an inlet channel 117, and an outlet channel 118. The drive mechanism 106 may further include a resilient sealing member 120 enclosing the pump chamber 115, and a first biasing device 160 (e.g., shape memory alloy (SMA) wire, an actuator such as an electro-osmotic actuator, or the like) operable to actuate or move a gearing mechanism and ultimately a plunger 124 relative to the pump chamber 115, as described in greater detail herein. In an example, the drive mechanism 106, the controller 109 and the drug reservoir 126 may be incorporated in a housing 105, which may be one or more parts that may be a combination of reusable and disposable parts. The housing 105 may be formed from one or more materials, such as a plastic, a metal or the like. The system 100 may include additional components not shown or described for the sake of brevity.

The controller 102 may receive a desired glucose level signal, which may be a first signal, indicating a desired glucose level or range for the patient 103. The desired glucose level or range may be stored in memory of a controller 109 on pump 104, received from a user interface of the controller 102, or another device, or by an algorithm within controller 109 (or controller 102). The sensor(s) 108 may be coupled to the patient 103 and operable to measure an approximate value of a glucose level of the user. In response to the measured glucose level or value, the sensor(s) 108 may generate a signal indicating the measured glucose value. As shown in the example, the controller 102 may also receive from the sensor(s) 108 via a communication path, the measured glucose level signal 112, which may be a second signal.

Based on the desired glucose level and the measured glucose level signal 112, the controller 102 or controller 109 may generate one or more control signals for directing operation of the pump 104. For example, one control signal 119 from the controller 102 or controller 109 may cause the pump 104 to turn on, or activate one or more power elements 123 operably connected with the pump 104. In the case where the first biasing device 160 is an SMA wire, activation of the SMA wire by the power element 123 may cause the SMA wire to change shape and/or length, which in turn may cause movement of the plunger 124 and the resilient sealing member 120. The specified amount of a liquid drug 125 (e.g., insulin, GLP-1, pramlintide, or a co-formulation of insulin, GLP-1 or pramlintide; a chemotherapy drug; a blood thinner; a pain medication; an arthritis drug; or the like) may be drawn from the reservoir 126 into the pump chamber 115, through the inlet channel 117, in response to a change in pressure due to the change in configuration of the resilient sealing member 120 and the plunger 124. In some examples, the specified amount of the liquid drug 125 to be delivered may be determined based on a difference between the desired glucose level and the actual glucose level signal 112. For example, specified amount of the liquid drug 125 may be determined as an appropriate amount of insulin to drive the measured glucose level of the user toward the desired glucose level. Based on operation of the pump 104, as determined by the control signal 119, the patient 103 may receive the liquid drug from a reservoir 126. The system 100 may operate as a closed-loop system, an open-loop system, or as a hybrid system. In an exemplary closed-loop system, the controller 109 may direct operation of the pump 104 without input from the user or controller 102, and may receive glucose level signal 112 from the sensor(s) 108. The sensor(s) 108 may be housed within the pump 104 or may be housed in a separate device and communicate wirelessly directly with the pump 104 (e.g., with controller 109) or with an external controller 102.

As further shown, the system 100 may include a needle deployment component 128 in communication with the controller 102 or the controller 109. Though shown separately, needle deployment component 128 may be integrated within pump 104. The needle deployment component 128 may include a needle and/or cannula 129 deployable into the patient 103 and may have one or more lumens and one or more holes at a distal end thereof. The cannula 129 may form a portion of a fluid path coupling the patient 103 to the reservoir 126. More specifically, the inlet channel 117 may be coupled to the reservoir 126 by a first fluid path component 130. The first fluid path component 130 may be of any size and shape and may be made from any material. The first fluid path component 130 enables fluid, such as the liquid drug 125 in the reservoir 126, to be transferred to the drive mechanism 106.

As further shown, the outlet channel 118 may be coupled to the cannula 129 by a second fluid path component 131. The second fluid path component 131 may be of any size and shape and may be made from any material. The second fluid path component 131 may be connected to the cannula 129 to allow fluid expelled from the pump 104 to be provided to the patient 103. The first and second fluid path components 130 and 131 may be rigid, flexible, or a combination thereof.

The controller 102/109 may be implemented in hardware, software, or any combination thereof. The controller 102/109 may, for example, be a processor, a logic circuit or a microcontroller coupled to a memory. The controller 102/109 may be a processor that includes a memory. The controller 102/109 may maintain a date and time as well as provide other functions (e.g., calculations or the like) performed by processors. The controller 102/109 may be operable to execute an artificial pancreas (AP) algorithm stored in memory (not shown in this example) that enables the controller 102/109 to direct operation of the pump 104. For example, the controller 102/109 may be operable to receive an input from the sensor(s) 108, wherein the input comprises analyte level data, such as glucose data or levels over time. Based on the analyte level data, the controller 102/109 may modify the behavior of the pump 104 and resulting amount of the liquid drug 125 to be delivered to the patient 103.

The power elements 123 may be one or more of a battery, a supercapacitor, a piezoelectric device, or the like, for supplying electrical power to the pump 104. In other embodiments, the power element 123, or an additional power source (not shown), may also supply power to other components of the pump 104, such as the controller 102, memory, the sensor(s) 108, and/or the needle deployment component 128.

In an example, the sensor(s) 108 may be a device communicatively coupled to the controller 102 and may be operable to measure a glucose value at a predetermined time interval, such as approximately every 5 minutes, 1 minute, or the like. The sensor(s) 108 may provide a number of glucose measurement values to the AP application.

In some embodiments, the pump 104, when operating in a normal mode of operation, provides insulin stored in the reservoir 126 to the patient 103 based on information (e.g., glucose measurement values, target glucose values, insulin on board, prior insulin deliveries, time of day, day of the week, inputs from an inertial measurement unit, global positioning system-enabled devices, Wi-Fi-enabled devices, or the like) provided by the sensor(s) 108 or other functional elements of the system 100 or pump 104. For example, the pump 104 may contain analog and/or digital circuitry that may be implemented at the controller 102/109 for controlling the delivery of the drug or therapeutic agent. The circuitry used to implement the controller 102/109 may include discrete, specialized logic and/or components, an application-specific integrated circuit, a microcontroller or processor that executes software instructions, firmware, programming instructions or programming code enabling, for example, an AP application stored in memory, or any combination thereof. For example, the controller 102/109 may execute a control algorithm and other programming code that may make the controller 102/109 operable to cause the pump to deliver doses of the drug or therapeutic agent to a user at predetermined intervals or as needed to bring glucose measurement values to a target glucose value. The size and/or timing of some of the doses may be pre-programmed, for example, into the AP application by the patient 103 or by a third party (such as a health care provider, a parent or guardian, a manufacturer of the wearable drug delivery device, or the like) using a wired or wireless link, or may be calculated iteratively by the controller 102 or controller 109, such as every 5 minutes.

Although not shown, in some embodiments, the sensor(s) 108 may include a processor, memory, a sensing or measuring device, and a communication device. The memory of the sensor(s) 108 may store an instance of an AP application as well as other programming code and be operable to store data related to the AP application.

In various embodiments, the processor of the sensor(s) 108 may include discrete, specialized logic and/or components, an application-specific integrated circuit, a microcontroller or processor that executes software instructions, firmware, programming instructions stored in memory, or any combination thereof.

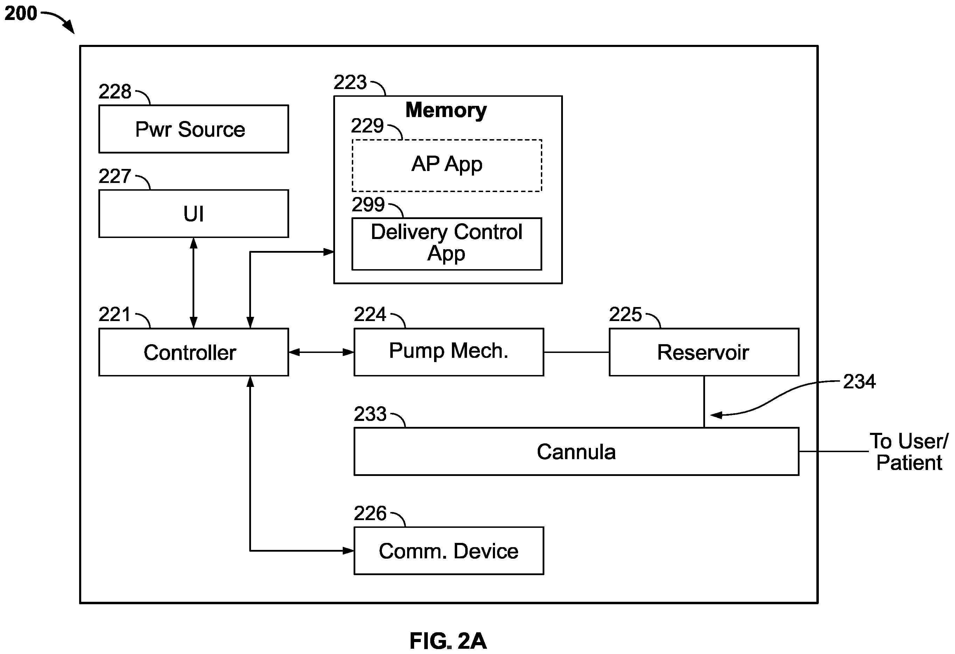

FIG. 2A illustrates a simplified block diagram of a drug delivery system 200. The drug delivery system 200 may include a controller 221, a memory 223, an AP application 229 and delivery control application 299 stored in the memory 223, a pump mechanism 224, a communication device 226, user interface 227, and a power source 228. The memory 223 may be operable to store programming code and applications including a delivery control application 299, the AP application 229, and data. The delivery control application 299 and the AP application 229 may optionally be stored on other devices.

The controller 221 may be coupled to the pump mechanism 224 and the memory 223. The controller 221 may include logic circuits, a clock, a counter or timer as well as other processing circuitry, and be operable to execute programming code and the applications stored in the memory 223 including the AP application 229 and/or the delivery control application 299. A communication device 226 may be communicatively coupled to the controller 221 and may be operable to wirelessly communicate with an external device, such as a personal diabetes management (PDM) device, a smart device such as a smartphone and/or a smartwatch, or the like.

For ease of discussion, the computer programs and computer applications that implement the medication delivery algorithms or applications may be referred to herein as an "AP application," a delivery control application," or an "automated drug delivery (ADD) algorithm."

The AP application 229 and/or the delivery control application 299 may be configured to provide automatic delivery of insulin based on an analyte sensor input, such as signals received from an analyte sensor, such as a continuous glucose monitor, or the like. The signals from the analyte sensor may contain glucose measurement values, timestamps, or the like. In addition, or alternatively, while examples may be described with reference to a closed loop algorithmic implementation, variations of the disclosed examples may be implemented to enable open loop use. The open loop implementations allow for use of different modalities of delivery of insulin such as smart pen, syringe, or the like in addition to the described drug delivery devices. For example, the disclosed AP application 229, the delivery control application 299, and related algorithms may be operable to perform various functions related to open loop operations, such as determination of a total daily setting for a drug or combination of drugs, such as a total daily insulin setting or the like.

The pump mechanism 224 may be operable to deliver a drug, like insulin, at a fixed or variable rate. For example, an AP application 229 or ADD algorithm executing on a personal diabetes management (PDM) device or a smart phone may determine or be informed that a user's total daily insulin (e.g., bolus and/or basal deliveries) is 48 units per 24 hours, which may translate to an exemplary physiological dosage rate of 1 unit per hour that may be determined according to a diabetes treatment plan. However, the pump mechanism 224 may be operable to deliver insulin at rates different from the example physiological dosage rate of 1 unit per hour. In an example, the system 200 may be attached to the body of a user, such as a patient or diabetic via, for example, by an adhesive, (e.g., directly attached to the skin of the user) and may deliver any therapeutic agent, including any drug or medicine, such as insulin, morphine, or the like, to the user. In an example, a surface of the system 200 may include an adhesive (not shown) to facilitate attachment to a user. The system 200 may, for example, be worn on a belt or in a pocket of the user and the liquid drug may be delivered to the user via tubing to an infusion site on the user.

In various examples, the drug delivery system 200 may be an automatic, wearable drug delivery device. For example, the drug delivery system 200 may include a reservoir 225 configured to hold a liquid drug (such as insulin), a needle and/or cannula 233 for delivering the drug into the body of the user (which may be done subcutaneously, intraperitoneally, or intravenously), and a pump mechanism 224, or other drive mechanism, for transferring the drug from the reservoir 225, through a needle or cannula 233, and into the user.

The pump mechanism 224 may be fluidly coupled to reservoir 225, and communicatively coupled to the medical device controller 221. The pump mechanism 224 may be coupled to the reservoir 225 and operable to output the liquid drug from the reservoir 225 via a fluid delivery path and out of the cannula 233. The pump mechanism 224 may have mechanical parameters and specifications, such as a pump resolution, that indicate mechanical capabilities of the pump mechanism. The pump mechanism 224 may also have electrical connections to control circuitry (not shown) that is operable to control operation of the pump mechanism 224. The pump resolution is a fixed amount of insulin the pump mechanism 224 delivers in a pump mechanism pulse, which is an actuation of the pump mechanism for a preset time period. Actuation may be when power from the power source 228 is applied to the control circuitry coupled to the pump mechanism 224 and the pump mechanism 224 operates to pump a fixed amount of insulin in a preset amount of time from the reservoir 225. Alternatively, the pump mechanism 224 may be substantially mechanical in structure and operation and utilize mechanical energy storage devices, such as springs or other biasing members to operate the pump mechanism 224.

The cannula 233 of FIG. 2A may be coupled to the reservoir 225 via a fluid delivery path 234. The cannula 233 may be operable to output the liquid drug to a user when the cannula 233 is inserted in the user.

The drug delivery system 200 may also include a power source 228, such as a battery, a supercapacitor, a piezoelectric device, or the like, that is operable to supply electrical power to the pump mechanism 224 and/or other components (such as the controller 221, memory 223, and the communication device 226) of the drug delivery system 200.

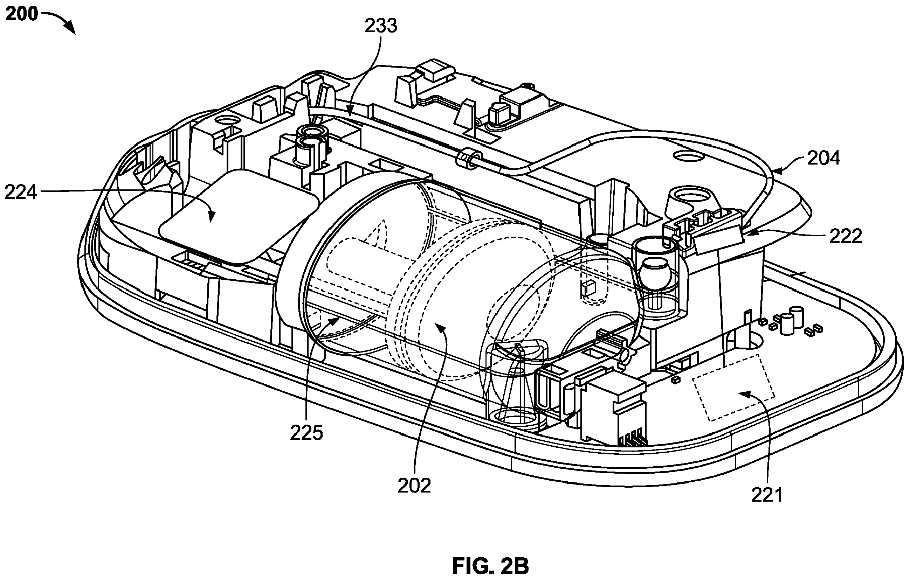

As shown in FIG. 2B, the drug delivery system 200 may include a plunger 202 positioned within the reservoir 225. An end portion or stem of the plunger 202 can extend outside of the reservoir 225. The pump mechanism 224 may, under control of the controller 221, be operable to cause the plunger 202 to expel the fluid, such as a liquid drug (not shown) from the reservoir 225 and into a fluid component 204 and cannula 233 by advancing into the reservoir 225. In various examples, a sensor, such as that shown at 222, may be integrated anywhere along the overall fluid delivery path of the drug delivery system 200, which includes the reservoir 225, the fluid delivery path component 204, and the cannula 233.

The controller 221 may be implemented in hardware, software, or any combination thereof. In various examples, the controller 221 can be implemented as dedicated hardware (e.g., as an application specific integrated circuit (ASIC)). The controller 221 may be a constituent part of the drug delivery system 200, can be implemented in software as a computational model, or can be implemented external to the drug delivery system 200 (e.g., remotely). The controller 221 may be configured to communicate with one or more sensors (e.g., sensor(s) 108 of FIG. 1 or sensor 222).

A reservoir, such as 225, may be included in a drug delivery device to store a liquid drug (e.g., insulin). For example, the reservoir 225 may be filled, or partially filled, with a liquid drug or a liquid drug solution. In one example, a liquid drug solution is a mixture of the liquid drug and added preservatives. The reservoir may store the liquid drug until all of the liquid drug has been dispensed (e.g., into a patient via a cannula). As such, the liquid drug (or solution) may remain in the reservoir for a period of time (e.g., 1 day, 3 days, 1 week, 2 weeks, etc.).

As discussed above, glucose concentration measurements may be used by automated insulin delivery (AID) systems to adjust subcutaneous deliveries provided to users. Such measurements may be provided via one or more subcutaneous sensors (e.g., sensor(s) 108 of FIG. 1). In some examples, glucose concentration measurements are received (or collected) by the ADD system periodically. Measurements may be provided over a plurality of control cycles of the ADD system. The control cycles may be evenly distributed in time. For example, if there are 12 cycles per hour, a new cycle may start every 5 minutes. Of course, the number of cycles per hour may differ (e.g., every minute) or vary (e.g., every minute during one period of time and every 10 minutes during a second period of time) over time.

However, there may be a delay between a rapid change in glucose concentration occurs and the time that the ADD system reacts to the change. For example, the rapid change may occur between control cycles. Likewise, variations in the life pattern of a user may cause the user's glucose concentration to vary in an unpredictable or unforeseeable manner. In such cases, it may take multiple glucose concentration measurements (e.g., across multiple control cycles) for the ADD system to determine an appropriate action. In some cases, the subcutaneous sensor(s) collecting the glucose concentration measurements can experience inherent delays that contribute to the lag effect between the occurrence of the rapid change and the reaction by the ADD system. In some cases, this delayed response by the ADD system can expose users to increased risks of hyperglycemia and hypoglycemia.

Accordingly, improved systems and methods for identifying changes in user glucose concentrations are provided herein. In at least one embodiment, a drug delivery system includes a user interface that allows the user to provide an indication of their perceived glucose state. The perceived glucose state of the user is evaluated using a glucose measurement value corresponding in time to when the user indication was received. A trust index is assigned to the user based on a result of the evaluation. In some examples, an adjustment to a drug delivery algorithm is determined based on the assigned trust index. The adjustment may be utilized in a determination of a drug delivery dosage to be administered to the user.

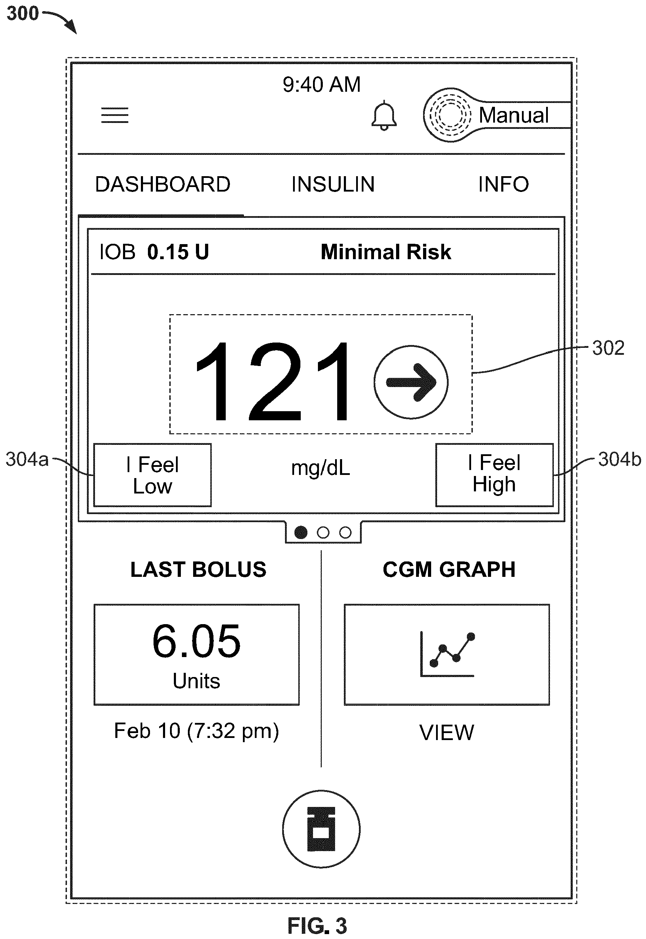

FIG. 3 illustrates a user interface (UI) 300 of a drug delivery system in accordance with aspects described herein. In one example, the UI 300 is provided by a user application running on a user device (e.g., a smartphone, a smartwatch, a PDM device, etc.). In some examples, the user application corresponds to the AP application 229 and/or the delivery control application 299 of FIG. 2.

As shown, the UI 300 includes a glucose measurement value 302. In one example, the glucose measurement value 302 represents the user's current (or most recent) glucose concentration. The glucose measurement value 302 may be updated whenever a new measurement is received at the user device (e.g., every control cycle). In some examples, the glucose measurement value 302 represents the measurements collected by one or more subcutaneous sensor(s) (e.g., CGM sensor(s), analyte sensor(s)). The subcutaneous sensor(s) may correspond to the sensor(s) 108 of FIG. 1.

In one example, the UI 300 includes a first indicator button 304a and a second indicator button 304b. The first indicator button 304a corresponds to a perceived user state of "I Feel Low" and the second indicator button 304b corresponds to a perceived user state of "I Feel High." As such, the user may select the first indicator button 304a when feeling "low" and the second indicator button 304b when feeling "high." The user may determine their own perceived state by evaluating how they feel, which may be relative to the glucose measurement value 302. For example, when the user believes their glucose concentration is actually lower than the glucose measurement value 302, the user may select the first indicator button 304a. Likewise, when the user believes their glucose concentration is actually higher than the glucose measurement value 302, the user may select the second indicator button 304a. In some examples, the user determines their own perceived state by evaluating how they feel relative to their preferred glucose concentration state (or the associated feeling). For example, when the user feels lower than their preferred glucose concentration state, the user may select the first indicator button 304a. Likewise, when the user feels higher than their preferred glucose concentration state, the user may select the second indicator button 304a. In some examples, the UI 300 presents a prompt requesting an input indicating the perceived glucose state of the user (e.g., the selection of an indicator button 304a or 304b). For example, the system may prompt the user via UI 300 for an input regarding the user's perceived glucose state when the system detects a rapid change in glucose concentration levels (e.g., as measured from one cycle to the next). A rapid change may be a change of 15mg/dL or more, for example, in the upward or downward direction.

While the UI 300 includes two indicator buttons 304a, 304b, it should be appreciated that the UI 300 may include a different number of indicator buttons. In one example, only one indicator button is provided (e.g., button 304a or 304b). In other examples, more than two indicator buttons are provided. For example, the UI 300 may include three indicator buttons corresponding to perceived glucose concentration states of: low, in range (or good), and high. In another example, the UI 300 may include four indicator buttons corresponding to states of: very low, low, high, and very high. In another example, the UI 300 may include six indicator buttons corresponding to states of: very low, low, low but in range, high but in range, high, and very high. Likewise, the indicators may correspond to specific glucose concentration states. For example, the UI 300 may include indicator buttons corresponding to: hypoglycemic (or hypo), in range, and hyperglycemic (or hyper). The indicator buttons may have different colors and/or icons corresponding to the relative risk of the representative state (e.g., red on the margins (e.g., very low or very high), yellow toward the center (e.g., low, high), and green in the center (e.g., in range, or good). The number of indicator buttons or the labeling of the indicator buttons may be specific to the user or may be specific to the device on which the UI is displayed (e.g., on a smartwatch, fewer elements may be displayed than on a smartphone). In some examples, the configuration of the indicator buttons corresponds to the experience level of the user. Newer (or younger) diabetics may be presented with fewer, simpler indicators compared to experienced (or older) diabetics. In some examples, the configuration of the indicator buttons may change dynamically based on the amount of trust afforded to the user by the user application.

In one example, a perceived state signal is generated each time the user selects the indicator buttons 304a, 304b. The perceived state signal represents the perceived glucose state of the user (e.g., high or low) at the time the button was selected. In some examples, the perceived state signal is provided to a glucose concentration evaluation engine of the user application. In other examples, the perceived state signal is provided to a different engine or module of the user application or to a separate application (e.g., a cloud application) in communication with the user application. The perceived state signal may be used to identify rapid changes in the user's glucose concentration and to adjust operation of the drug delivery system, as described in greater detail below.

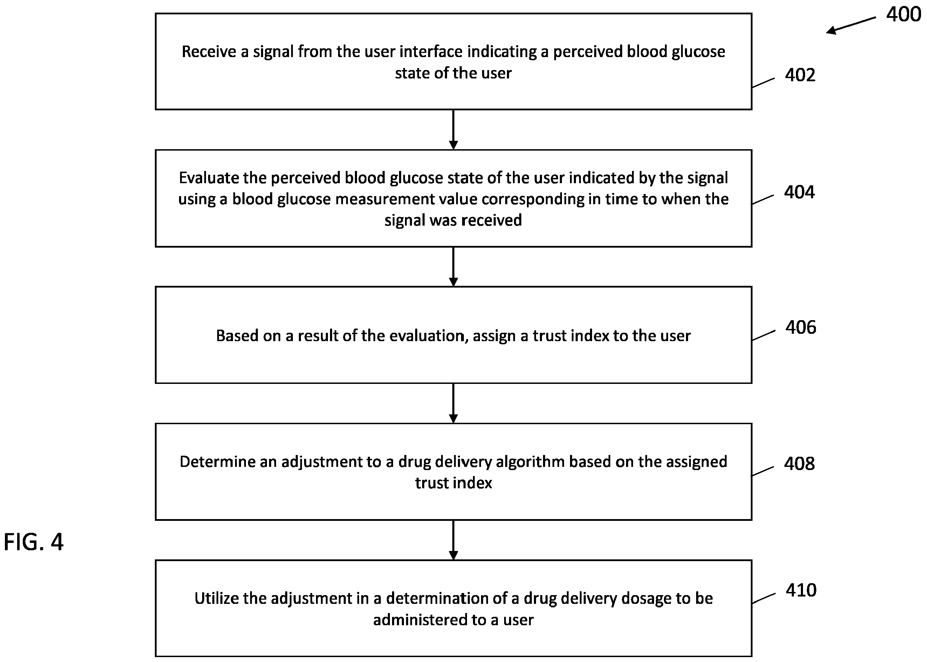

FIG. 4 illustrates a flow diagram of an example method for evaluating a user's perceived glucose state and assigning a trust index to the user based on the evaluation. In one example, a drug delivery algorithm is adjusted based on the trust index. In some examples, the adjusted drug delivery algorithm is used to determine a drug delivery dosage administered to the user.

The method 400 may be performed, at least in part, by the glucose concentration evaluation engine or a different engine of the user application (e.g., the AP application 229 and/or the delivery control application 299 of FIG. 2). In some examples, the method 400 may be performed, at least in part, by a controller or a processor of an ADD device (e.g., controller 102 or controller 109 of FIG. 1 or controller 221 of FIG. 2). In other examples, the method 400 can be performed by controllers, processors, or computing systems that are external to the drug delivery system.

At block 402, a perceived state signal is received from the UI 300 indicating a perceived glucose state of the user. As described above, the perceived state signal is generated by the user application in response to the user selecting one of the indicator buttons 304a, 304b. The perceived state signal provides an indication that the user is feeling "high" or "low" at a particular time. In some examples, the perceived state signal is received by the glucose concentration evaluation engine of the user application; however, in other examples, the perceived state signal may be received by a different engine, controller, processor, etc.

At block 404, the perceived glucose state of the user (as indicated by the perceived state signal) is evaluated using a glucose measurement value. In one example, the glucose measurement value corresponds in time to when the perceived state signal is received. For example, the glucose measurement value may correspond to the glucose measurement value 302 at the time the perceived state signal is received. In some examples, the glucose measurement value is the measurement value 302 received during the most recent control cycle (e.g., prior to receipt of the perceived state signal). If the perceived state signal is received between control cycles, the user application may wait until a new measurement value 302 is received at the next control cycle. In other examples, if the perceived state signal is received between control cycles, an average of the measurement values 302 from the control cycles immediately prior to and after receipt of the perceived state signal may be used as the glucose measurement value.

At block 406, a trust index is assigned to the user based on a result of the evaluation. In one example, the trust index provides an indication of the user's reliability in assessing their own glucose state. Each type of perceived glucose state may be evaluated differently. The user may have a first trust index indicating their reliability in assessing a perceived low glucose state and a second trust index indicating their reliability in assessing a perceived high glucose state. For example, Equations (1a) and (1b) below may be used to evaluate a perceived low glucose state (e.g., in response to the user selecting the first indicator button 304a):

$$T_{Low} = w + \frac{\sum_{l=1}^{N} \sum_{j=1}^{R} B_l(j)}{N} \qquad \text{Equation (1a)}$$

$$B_l(j) = \begin{cases} 0.125 & G_B(b+j) \leq 70 \\ 0.0675 & 70 < G_B(b+j) \leq 100 \\ 0 & 100 < G_B(b+j) \end{cases} \qquad \text{Equation (1b)}$$

where, $T_{Low}$ is a low glucose trust index of the user, w is a biasing value, $l$ is a count of button presses (e.g., of the first indicator button 304a), $N$ is a total number of button presses, $j$ is a count of control cycles since the last button press, $R$ is a total number of control cycles, $B_l(j)$ is a trust increment value, $G_B$ is a measured glucose concentration of the user, and $b$ is the control cycle in which the button was pressed by the user. In one example, each control cycle is 5 minutes; however, different control cycle durations may be used. In some examples, 10 different button presses may be used to determine $T_{Low}$ (e.g., $N$=10); however, a different number of button presses may be used. In one example, the trust index $T_{Low}$ has a value between $w$ and 1, with $w$ being the lowest level of trust and 1 being the highest level of trust. Of course, the values of 0.125, 0.0675, 70 and 100 mentioned in equation 1b above are only specific exemplary values. It should be understood that other values in a variation of up to 25% (compared to the specifically mentioned values), more specifically by up to 20% or up to 10% may be used.

Likewise, Equations (2a) and (2b) below may be used to evaluate a perceived high glucose state (e.g., in response to the user selecting the second indicator button 304b):

$$T_{High} = \frac{\sum_{h=1}^{N1} \sum_{j=1}^{R1} B_h(j)}{N1} \qquad \text{Equation (2a)}$$

$$B_h(j) = \begin{cases} 0.25 & 180 < G_B(b+j) \\ 0.0125 & 140 < G_B(b+j) \leq 180 \\ 0 & G_B(b+j) \leq 140 \end{cases} \qquad \text{Equation (2b)}$$

where, $T_{High}$ is a high glucose trust index of the user, $h$ is a count of button presses (e.g., of the second indicator button 304b), $N1$ is a total number of button presses, $j$ is a count of control cycles since the last button press, $R1$ is a total number of control cycles, $B_h(j)$ is a trust increment value, $G_B$ is a measured glucose concentration of the user, and $b$ is the control cycle in which the button was pressed by the user. In one example, each control cycle is 5 mins; however, different control cycle durations may be used. In some examples, 10 different button presses are used to determine $T_{High}$ (e.g., $N1$=10); however, a different number of button presses may be used. In one example, the trust index $T_{high}$ has a value between 0 and 1, with 0 being the lowest level of trust and 1 being the highest level of trust. Again, the values of 0.25, 0.0125, 140 and 180 mentioned in equation 2b above are only specific exemplary values. It should be understood that other values in a variation of up to 25% (compared to the specifically mentioned values), more specifically by up to 20% or up to 10% may be used.

At block 408, an adjustment to a drug delivery algorithm is determined based on the assigned trust index (or indices). In one example, the trust index (or indices) is used to adjust the aggressiveness of the drug delivery algorithm. For example, Equations (3a), (3b), and (3c) below may be used to independently modify the relative penalization for glucose excursions below versus above the user's target glucose concentration:

$$J_{quad}(i) = \sum_{j=1}^{P} Q_{Low} \left( G_{\leq SP(j)}(j) - SP(j) \right)^2 + Q_{High} \left( G_{>SP(j)}(j) - SP(j) \right)^2 + \qquad \text{Equation (3a)}$$

$$R \left( I(j) - B(j) \right)^2$$

$$Q_{Low} = Q_{Low,base}(1 + T_{Low}) \qquad \text{Equation (3b)}$$

$$Q_{High} = Q_{High,base}(1 + T_{High}) \qquad \text{Equation (3c)}$$

where, $J_{quad}(i)$ is a quadratic cost function corresponding to the overall cost of a specific projected glucose and insulin delivery trajectory that would be minimized in an optimization function. $P$ is the prediction horizon over which the cost can be calculated (e.g., a number of control cycles). $Q_{Low}$ and $Q_{high}$ are coefficients that penalize glucose excursions $G(j)$ against the setpoint $SP(j)$ in the $j^{th}$ control cycle. For example, the coefficient $Q_{Low}$ is applied when the user's glucose level is equal to or less than the setpoint $SP(j)$ and the coefficient $Q_{high}$ is applied when the user's glucose level is greater than the setpoint $SP(j)$. $R$ is a coefficient that penalizes the insulin delivery $I(j)$ of the projected trajectory against a current user basal profile $B(j)$. The values for $Q_{Low}$, $Q_{high}$, and $R$ depend on the ratio between the penalization of glucose and insulin excursions that may be applied for the specific ADD algorithm. In some examples, the values for $Q_{Low}$, $Q_{high}$, and $R$ are tunable parameters. In some examples, insulin excursion may be calculated in units of insulin, and glucose excursion may be calculated in mg/dL. In such examples, a typical value for $Q_{Low}$ may be 1/1000 whereas a typical value for $Q_{high}$ may be 1/9000. It should be appreciated that both values $Q_{Low}$, $Q_{high}$, may range from 1/100 to 1/100,000. In some examples, the coefficient R is set to 1.

In some examples, the trust index (or indices) is utilized to modify the user's target glucose concentrations. In one embodiment, the user's target glucose can potentially be modified by at most 20 mg/dL, as shown in Equation (4):

$$SP_{new} = max\left(100, \min\left(SP_{base}(k) + 20(T_{Low}) - 20(T_{High}), 150\right)\right) \qquad \text{Equation (4)}$$

where $SP_{new}$ is the new target glucose for the user (e.g., setpoint), which is adjusted based on the user's current trust indices $T_{low}$ and $T_{high}$, respectively. $SP_{base}$ indicates the user's standard target glucose concentration prior to this modification. In some examples, $SP_{base}$ is a tunable parameter dependent on the specific ADD algorithm. In some examples, the value of $SP_{base}$ may range from 100 to 150 mg/dL. The ' 100' and '150' parameters are exemplary minimum and maximum adjustments that may be made to the target glucose, and may reference the limits of ranges in target glucose that have been demonstrated to be safe to the ADD algorithm. The '20' coefficients for $T_{Low}$ and $T_{High}$ may represent the adjustments that can be made at the maximum trust level for the user expressing hypoglycemia or hyperglycemia. It should be appreciated that the same coefficients may range anywhere from 0 to 50, or the maximum differences in the minimum and maximum target glucose concentrations. A higher value for this coefficient parameter may indicate a higher dependence by the adjusted target glucose concentration value on the trust indices for the user's hyperglycemia or hypoglycemia indications.

At block 410, the adjustment to the drug delivery algorithm is utilized to determine a drug delivery dosage to be administered to a user. The ADD device may be controlled to deliver insulin to the user according to the determined drug delivery dosage. In one example, the ADD device is controlled such that drug is expelled from a drug reservoir of the ADD device at a drug delivery rate corresponding to the determined drug delivery dosage. In some examples, the insulin delivery rate corresponds to one or more control signals that control one or more components of the ADD device. For example, a drive mechanism having a fluid pathway coupled to the reservoir may be operable to receive the control signal(s) and expel an amount of drug from the reservoir based on the received control signal(s) (e.g., to administer the determined drug delivery dosage).

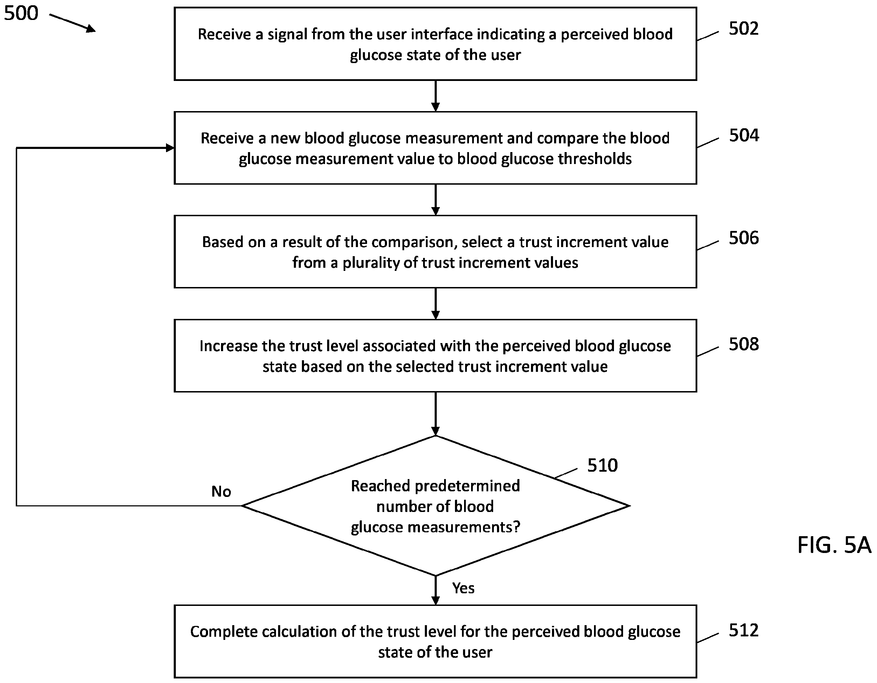

FIG. 5A is a flow diagram of an example method for determining a trust level associated with each button press performed by the user. In other words, the method 500 may be performed to determine a trust level associated with each perceived glucose state of the user (e.g., "I Feel High" or "I Feel Low"). In one example, the method 500 is performed using at least a portion of Equations (1a), (1b) or Equations (2a), (2b).

The method 500 may be performed, at least in part, by the glucose concentration evaluation engine or a different engine of the user application (e.g., the AP application 229 and/or the delivery control application 299 of FIG. 2). In some examples, the method 500 may be performed, at least in part, by a controller or a processor of an ADD device (e.g., controller 102 or controller 109 of FIG. 1 or controller 221 of FIG. 2). In other examples, the method 500 can be performed by controllers, processors, or computing systems that are external to the drug delivery system.

At block 502, a perceived state signal is received from the UI 300 indicating a perceived glucose state of the user. As described above, the perceived state signal is generated by the user application in response to the user selecting one of the indicator buttons 304a, 304b. The perceived state signal provides an indication that the user is feeling "high" or "low" at a particular time.

For exemplary purposes, the remaining steps of method 500 will be described with respect to a user selection of the first indicator button 304a (i.e., a perceived low glucose state).

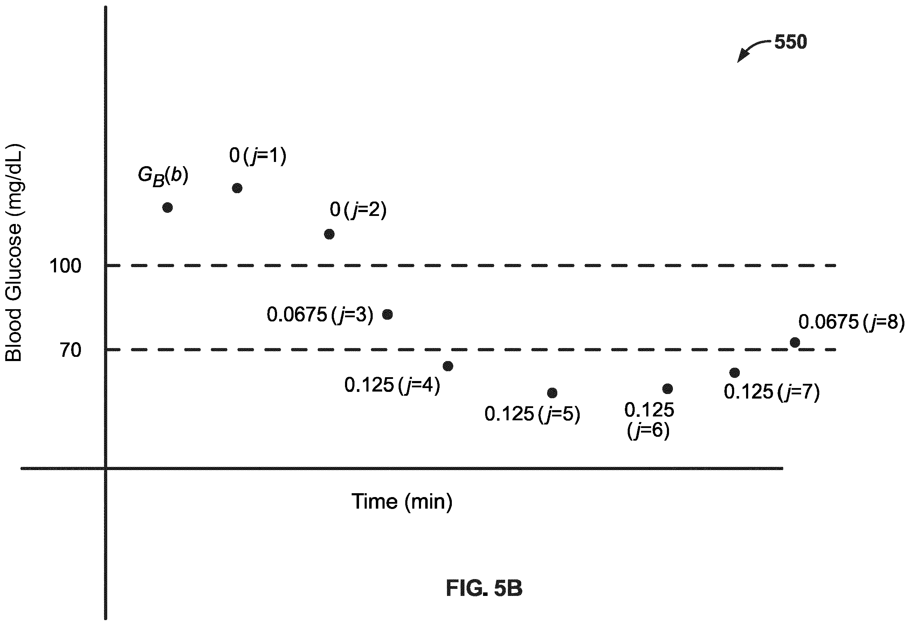

FIG. 5B illustrates an example series of glucose measurements $G_B$ received after the user selection of the first indicator button 304a. The glucose measurement $G_B(b)$ indicates the user's glucose during the control cycle in which the first indicator button 304a was selected. The subsequent glucose measurements $G_B(b + j)$ represent the user's glucose over time following the selection of the first indicator button 304a.

At block 504, a new glucose measurement $G_B(b + j)$ is received and compared to glucose thresholds. In one example, the glucose measurement $G_B(b + j)$ received corresponds to the user's measured glucose during the next control cycle (e.g., $G_B(b + 1)$, $G_B(b + 2)$, etc.). Since the perceived state signal received in block 502 indicates that the user is feeling low, the glucose measurement $G_B$ is compared to the thresholds in Equation (1b) (e.g., 70 mg/dL and 100 mg/dL).

At block 506, a trust increment value is selected from a plurality of trust increment values based on a result of the comparison. In one example, the trust increment value is selected based on how closely the perceived glucose state of the user matches the glucose measurement value obtained since the perceived state signal was received.

In some examples, the glucose measurement value is compared to various glucose thresholds and the trust increment value is selected from a plurality of trust increment values based on a result of the comparison. For example, since the user is feeling low, a trust increment value is selected from the trust increment values in Equation (1b). In one example, the trust increment value of 0.125 is selected if the glucose measurement $G_B$ is less than 70 mg/dL, the trust increment value of 0.0675 is selected if the glucose measurement $G_B$ is greater than 70 mg/dL but less than or equal to 100 mg/dL, and the trust increment value of 0 is selected if the glucose measurement $G_B$ is greater than 100 mg/dL. For example, as shown in FIG. 5B, the user's glucose measurement during control cycle $j$=1 is greater than 100 mg/dL. As such, the trust increment value of 0 is selected if the glucose measurement $G_B(b + 1)$. Likewise, the user's glucose measurement during control cycle $j$=3 is greater than 70 mg/dL and less than 100 mg/dL, corresponding to a trust level increment of 0.0675. Similarly, the user's glucose measurement during control cycle $j$=4 is less than 70 mg/dL, corresponding to a trust level increment of 0.125. It should be appreciated that different trust increment values may be contemplated.

At block 508, the trust level associated with the user's perceived glucose state is increased based on the selected trust increment value. For example, the trust level after the control cycle $j$=1 is 0, the trust level after the control cycle $j$=2 is 0, the trust level after the control cycle $j$=3 is 0.0675, the trust level after the control cycle $j$=4 is 0.1925 (i.e., 0.0675 + 0.125), and so on.

At block 510, it is determined whether a predetermined number of glucose measurements has been reached. The predetermined number of glucose measurements may correspond to the total number of control cycles $R$ in Equation (1a). In other words, glucose measurements are collected or received until $j$=$R$. For example, if $R$=8, the trust level corresponds to 8 different glucose measurements from 8 different control cycles. In response to a determination that the predetermined number of glucose measurements has not been reached, the method 500 returns to block 504 and continues to increment the trust level until $j$=$R$.

At block 512, in response to a determination that the predetermined number of glucose measurements has been reached (e.g., $j$=$R$), the calculation of the trust level for the perceived glucose state of the user is completed. The trust level represents the sum of the trust level increments selected for each of the received glucose measurements (e.g., $G_B(b + 1)$, $G_B(b + 2)$, etc.). For example, the trust level for the example shown in FIG. 5B is 0.635 out of 1, or 63.5%. As described above, the trust level represents the user's ability to perceive their own glucose state with respect to a single occurrence (e.g., selection of first indicator button 304a).

While the example above describes determining a trust level for a perceived low glucose state, a trust level for a perceived high glucose state may be determined similarly. For example, if the perceived state signal received in block 502 indicates that the user is feeling high, the glucose measurements $G_B$ are compared to the thresholds in Equation (2b) (block 504). Likewise, the trust increment values are selected from the trust increment values in Equation (2b) (block 506). In one example, the trust increment value of 0.25 is selected if the glucose measurement $G_B$ is greater than 180 mg/dL, the trust increment value of 0.0125 is selected if the glucose measurement $G_B$ is greater than 140 mg/dL but less than or equal to 180 mg/dL, and the trust increment value of 0 is selected if the glucose measurement $G_B$ is less than 140 mg/dL. It should be appreciated that different trust increment values may be contemplated. As described above, the trust level is incrementally increased with the selected trust increment values for each glucose measurement $G_B$ (block 508) until a predetermined number of glucose measurements has been reached (block 510). In one example, the predetermined number of glucose measurements corresponds to the total number of control cycles $R1$ in Equation (2a). In some examples, a smaller number of control cycles are used when determining the trust level for a perceived high glucose state (e.g., $R > R1$), tightening the requirements for impacting the trust level (e.g., to reduce risk of insulin over-delivery). Once the predetermined number of glucose measurements has been reached (e.g. $j$=$R1$), the calculation of the trust level for the perceived glucose state of the user is completed.

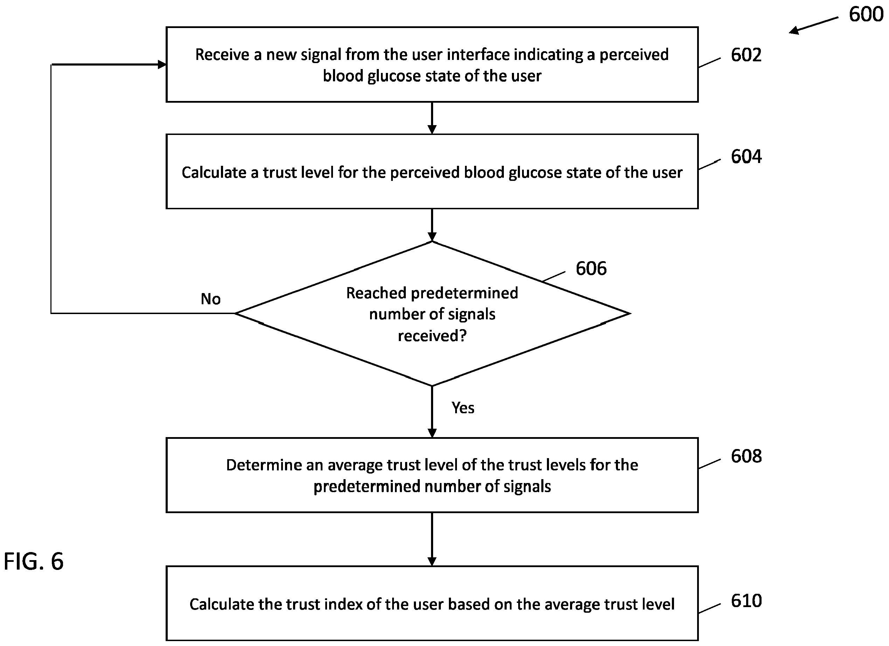

FIG. 6 is a flow diagram of an example method for determining a trust index associated with the user. In other words, the method 600 may be performed to determine a trust index associated with the user's ability to perceive their own glucose state (e.g., "I Feel High" or "I Feel Low"). In one example, the method 600 is performed using at least a portion of Equations (1a), (1b) or Equations (2a), (2b).

At block 602, a perceived state signal is received from the UI 300 indicating a perceived glucose state of the user. As described above, the perceived state signal is generated by the user application in response to the user selecting one of the indicator buttons 304a, 304b. The perceived state signal provides an indication that the user is feeling "high" or "low" at a particular time.

At block 604, a trust level is calculated for the perceived glucose state of the user. In one example, the trust level is calculated using the method 500 of FIG. 5A. As described above, the trust level represents the user's ability to perceive their own glucose state with respect to a single occurrence (e.g., a single selection of indicator buttons 304a, 304b).

At block 606, it is determined whether a predetermined number of perceived state signals has been reached. In other words, it is determined whether a predetermined number of trust levels has been calculated. For example, when determining the low glucose trust index of the user $T_{Low}$, the predetermined number of perceived state signals (or calculated trust levels) corresponds to the total number of button presses $N$ in Equation (1a). In other words, signals indicating a perceived low glucose state are received until $l=N$. Likewise, when determining the high glucose trust index of the user $T_{High}$, the predetermined number of perceived state signals (or calculated trust levels) corresponds to the total number of button presses $N1$ in Equation (2a). In other words, signals indicating a perceived high glucose state are received until $h=N1$. In response to a determination that the predetermined number of perceived state signals has not been reached, the method 600 returns to block 602 and continues to calculate trust levels (block 604).

At block 608, in response to a determination that the predetermined number of perceived state signals has been reached (e.g., $l=N$ or $h=N1$), an average trust level is determined. For example, as shown in Equation (1a), the trust levels calculated for each button press of the first indicator button 304a (e.g., "I Feel Low") are summed and divided by the predetermined number of button presses $N$. The result is an average trust level across the $N$ series of perceived low glucose states. Likewise, as shown in Equation (2a), the trust levels calculated for each button press of the second indicator button 304b (e.g., "I Feel High") are summed and divided by the predetermined number of button presses $N1$. The result is an average trust level across the $N1$ series of perceived high glucose states.

At block 610, the trust index is calculated based on the determined average trust level. As shown in Equation (1a), to calculate the low glucose trust index $T_{Low}$, a biasing value w is added to the average trust level. In some examples, the addition of the biasing value w may be optional. As shown in Equation (1b), the high glucose trust index $T_{High}$ corresponds to the average trust level. By not including a biasing value, stricter requirements are applied to the high glucose trust index $T_{High}$ to reduce the risk of insulin over-delivery. In other examples, a biasing value may be added to the average trust level when calculating the trust index $T_{High}$.

While the examples described above use a predetermined number of button presses (e. g., $N$ or $N1$) to determine the trust indices $T_{Low}$ and $T_{High}$, it should be appreciated that the number of button presses used may be dynamic. For example, confirmatory fingersticks may reduce the number of button presses used to determine the trust indices. In one example, a weighted average is used to determine the trust indices where button presses having an associated confirmatory fingerstick are weighed more heavily than button presses not having an associated confirmatory fingerstick (e.g., twice as heavily). In such examples, the last $N-X$ (or $N1-X$) button presses may be used to determine the trust indices, where X is the number of button presses with confirmatory fingersticks. The value of the fingerstick glucose measurement may be utilized to confirm (or validate) the user's interactions. For example, if the user presses the first indicator button 304a (e.g., "I Feel Low"), a fingerstick measurement below 70 mg/dL that accompanies the button press may be considered confirmatory. Likewise, if the user presses the second indicator button 304b (e.g., "I Feel High"), a fingerstick measurement above 180 mg/dL that accompanies the button press may be considered confirmatory.

As described above, the trust indices $T_{Low}$ and $T_{High}$ automatically adapt to the accuracy of the user's last $N$ or $N1$ button presses (or the weighted equivalent). All user interactions (e.g., button presses) that occur within the span of one control cycle (e.g., 5 minutes) may be considered to be one interaction. In some cases, the indicator buttons 304a, 304b may be grayed out (or disabled) until the next interaction can be received (e.g., after a trust level has been calculated for the previous interaction). Alternatively, a user may be able to change their selection by selecting another indicator, and the indicator selected as of the end of the particular control cycle may be deemed to be the user's selection for that control cycle. In other examples, interactions that occur within a span of $Y$ control cycles (e.g., 2, 4, or 6 control cycles) are considered to be one interaction. In the event the user attempts to interact with both indicator buttons 304a, 304b during the same control cycle (or span of Y control cycles), the user's selection of the first indicator button 304a (e.g., "I Feel Low") may be prioritized.

The trust indices $T_{Low}$ and $T_{High}$ may be valid (or active) for a customizable number of control cycles or window of time (e.g., 30 mins, 1 hr, 1 day, etc.). In one example, while active, the trust indices are used to adjust a drug delivery algorithm (e.g., block 408 of FIG. 4). Once the number of control cycles (or window of time) expires, the trust indices are made inactive. In one example, the trust indices have a value of 0 when inactive. In some examples, the trust indices are configured to decay over time. For example, the trust indices may decay exponentially as shown in Equations (5a) and (5b) below:

$$T_{Low,final} = T_{Low,base}\, e^{-\frac{j}{Z\ln(d)}} \qquad\qquad \text{Equation (5a)}$$

$$T_{High,final} = T_{High,base}e^{-\frac{j}{Z1\ln(d1)}}$$

Equation (5b)

where, $T_{Low, final}$ is the ending (or fully decayed) value of $T_{Low}$, $T_{Low, base}$ is the starting value of $T_{Low}$ (e.g., as determined from method 600 of FIG. 6), Z is the number of control cycles that $T_{Low}$ decays over, and d is the decay factor. In one example, if d=2, $T_{Low, final}$ will be half the value of $T_{Low, base}$ after decaying over Z control cycles. Likewise, $T_{High, final}$ is the ending (or fully decayed) value of $T_{High}$, $T_{High, base}$ is the starting value of $T_{Low}$ (e.g., as determined from method 600 of FIG. 6), Z1 is the number of control cycles that $T_{Low}$ decays over, and d1 is the decay factor. In one example, the values of Z and Z1 are the same; however, in other examples, the values may be different. In one example, the values of d and d1 are the same; however, in other examples, the values may be different.

In some examples, the number of control cycles Z or Z1 that the trust indices decay over corresponds to the window of time that the trust indices are active (or valid). In other examples, the trust indices may retain their full value during the active window of time and begin to decay (e.g., over Z or Z1 control cycles) once the active window of time has expired.

In one example, the trust indices calculated for the user are stored (e.g., in memory). The previous trust indices may be analyzed to determine a pattern of trust associated with the user. For example, the ADD system may afford more trust to a user who has demonstrated a pattern of high trust indices. In some examples, if the user's previous interactions had high trust indices (e.g., the user was reliable), the user's subsequent indications of "feeling high" or "feeling low" may be applied with an increased impact to the system's response to the user in magnitude, immediacy, or both. In some examples, if a user with a consistently high trust index in previous interactions begins demonstrating low trust indices, potential system issues may be identified. For example, given the trust indices incorporate confirmatory glucose readings, a sudden shift in the user's trust index patterns may indicate a glucose sensor issue, rather than a sudden change in the user's behavioral patterns.

As described above, improved system and methods for identifying changes in user glucose concentrations are provided herein. In at least one embodiment, a drug delivery system includes a user interface that allows the user to provide an indication of their perceived glucose state. The perceived glucose state of the user is evaluated using a glucose measurement value corresponding in time to when the user indication was received. A trust index is assigned to the user based on a result of the evaluation. In some examples, an adjustment to a drug delivery algorithm is determined based on the assigned trust index. The adjustment may be utilized in a determination of a drug delivery dosage to be administered to the user.

The techniques described herein for a drug delivery system (e.g., the system 100, the system 200, or any components thereof) may be implemented in hardware, software, or any combination thereof. Any component as described herein may be implemented in hardware, software, or any combination thereof. For example, the systems 100, 200 or any components thereof may be implemented in hardware, software, or any combination thereof. Software related implementations of the techniques described herein may include, but are not limited to, firmware, application specific software, or any other type of computer readable instructions that may be executed by one or more processors. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

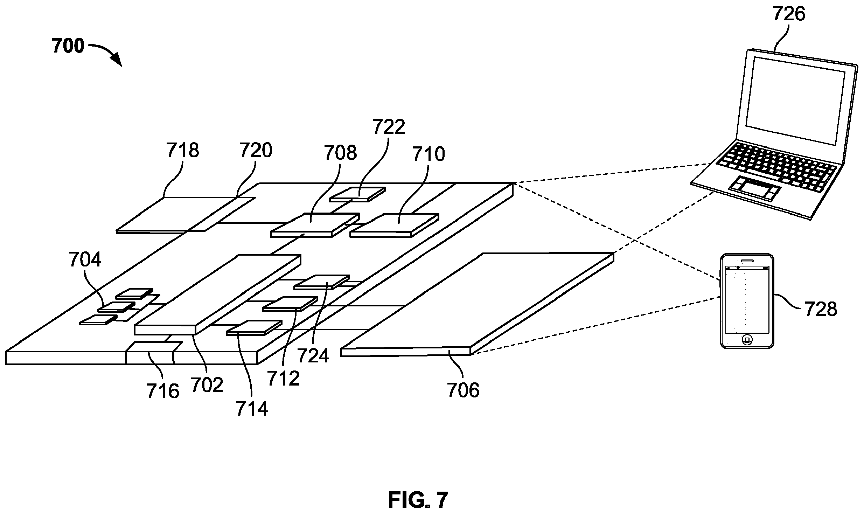

FIG. 7 shows an example of a generic computing device 700, which may be used with some of the techniques described in this disclosure (e.g., systems 100, 200 or devices in communication with the systems 100, 200). Computing device 700 may include a processor 702, a memory 704, an input/output device such as a display 706, a communication interface 708, and a transceiver 710, among other components. The device 700 may also be provided with an electronic storage device, such as a micro-drive or other device, to provide additional data and programming code storage. Each of the components 700, 702, 704, 706, 708, and 710, are interconnected using various buses, and several of the components may be mounted on a common motherboard or in other manners as appropriate.

The processor 702 can execute instructions within the computing device 700, including instructions stored in the memory 704. The processor 702 may be implemented as a chipset of chips that include separate and multiple analog and digital processors. The processor 702 may provide, for example, for coordination of the other components of the device 700, such as control of user interfaces, applications run by device 700, and wireless communication by device 700.

Processor 702 may communicate with a user through control interface 712 and display interface 714 coupled to a display 706. The display 706 may be, for example, a TFT LCD (Thin-Film-Transistor Liquid Crystal Display) or an OLED (Organic Light Emitting Diode) display, touchscreen display, or other appropriate display technology. The display interface 714 may comprise appropriate circuitry for driving the display 706 to present graphical and other information to a user. The control interface 712 may receive commands from a user and convert them for submission to the processor 702. In addition, an external interface 716 may be provided in communication with processor 702, so as to enable near area communication of device 700 with other devices. External interface 716 may provide, for example, for wired communication in some implementations, or for wireless communication in other implementations, and multiple interfaces may also be used.

The memory 704 stores information within the computing device 700. The memory 704 can be implemented as one or more of a computer-readable medium or media, a volatile memory unit or units, or a non-volatile memory unit or units. Expansion memory 718 may also be provided and connected to device 700 through expansion interface 720.

Device 700 may communicate wirelessly through communication interface 708, which may include digital signal processing circuitry where necessary. Communication interface 708 may in some cases be a cellular modem. Such communication may occur, for example, through radio-frequency transceiver 710. In addition, short-range communication may occur, such as using a Bluetooth, Wi-Fi, or other such transceiver (not shown). In addition, GPS (Global Positioning System) receiver module 722 may provide additional navigation- and location-related wireless data to device 700, which may be used as appropriate by applications running on device 700.

Device 700 may also communicate audibly using audio codec 724, which may receive spoken information from a user and convert it to usable digital information. Audio codec 724 may likewise generate audible sound for a user, such as through a speaker, e.g., in a handset of device 700.

The computing device 700 may be implemented in a number of different forms, as shown in FIG. 7. For example, it may be implemented as a computer (e.g., laptop) 726. It may also be implemented as part of a smartphone (or PDM device) 728, smart watch, tablet, personal digital assistant, or another similar mobile device.

While this specification contains many specific implementation details, these should not be construed as limitations on the scope of any inventions or of what may be claimed, but rather as descriptions of features specific to particular implementations of particular inventions. Certain features that are described in this specification in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the implementations described above should not be understood as requiring such separation in all implementations, and it should be understood that the described program components and systems can generally be integrated together in a single software product or packaged into multiple software products.

Thus, particular implementations of the subject matter have been described. Other implementations are within the scope of the following claims. In some cases, the actions recited in the claims can be performed in a different order and still achieve desirable results. In addition, the processes depicted in the accompanying figures do not necessarily require the particular order shown, or sequential order, to achieve desirable results.

Certain examples of the present disclosed subject matter were described above. It is, however, expressly noted that the present disclosed subject matter is not limited to those examples, but rather the intention is that additions and modifications to what was expressly described herein are also included within the scope of the disclosed subject matter. Moreover, it is to be understood that the features of the various examples described herein were not mutually exclusive and may exist in various combinations and permutations, even if such combinations or permutations were not made express herein, without departing from the spirit and scope of the disclosed subject matter. In fact, variations, modifications, and other implementations of what was described herein will occur to those of ordinary skill in the art without departing from the spirit and the scope of the disclosed subject matter. As such, the disclosed subject matter is not to be defined only by the preceding illustrative description.

Program aspects of the technology may be thought of as "products" or "articles of manufacture" typically in the form of executable code and/or associated data that is carried on or embodied in a type of machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of example examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

In particular, although the present disclosure is defined in the enclosed claims and has been described above, it should be understood that the present disclosure can alternatively be defined in accordance with the following embodiments:

1. A drug delivery system, comprising:

at least one memory storing programming code operable to control delivery of a drug;
a user interface operable to accept an input indicating a perceived glucose state of a user; and
at least one processor operable to execute the programming code, wherein the programming code causes the processor to:

receive a perceived state signal from the user interface indicating the perceived glucose state of the user;
evaluate the perceived glucose state of the user indicated by the perceived state signal using a glucose measurement value corresponding in time to when the perceived state signal was received;
determine an adjustment to a drug delivery algorithm; and
utilize the adjustment in a determination of a drug delivery dosage to be administered to a user.

2. The drug delivery system of embodiment 1, wherein the programming code further causes the processor to: based on a result of the perceived glucose state evaluation, assign a trust index to the user.

3. The drug delivery system of embodiment 1 or 2, wherein the programming code further causes the processor, when evaluating the perceived glucose state of the user indicated by the perceived state signal, to: select a trust increment value based on how closely the perceived glucose state of the user matches the glucose measurement value obtained since the perceived state signal was received.

4. The drug delivery system of one of the preceding embodiments, wherein the programming code further causes the processor, when selecting the trust increment value, to: compare the glucose measurement value to various glucose thresholds;
based on a result of the comparison, select the trust increment value from a plurality of trust increment values.

5. The drug delivery system of one of the preceding embodiments, wherein the selected trust increment value is an indicator of the user's reliability in perceiving their own glucose state.

6. The drug delivery system of one of the preceding embodiments, wherein the programming code further causes the processor, to: calculate the trust index by increasing a trust level associated with the perceived glucose state based on the selected trust increment value.

7. The drug delivery system of embodiment 6, wherein the trust level represents a sum of trust increment values selected for a predetermined number of glucose measurement values obtained following receipt of the perceived state signal.

8. The drug delivery system of embodiment 7, wherein the programming code further causes the processor, when calculating the trust index, to:

calculate trust levels for a predetermined number of perceived state signals received from the user interface; and
determine an average trust level of the trust levels for the predetermined number of perceived state signals.

9. The drug delivery system of embodiment 8, wherein the predetermined number of perceived state signals received from the user interface correspond to previous perceived state signals that indicate the user's perceived glucose state is lower than a low glucose setpoint.

10. The drug delivery system of embodiment 8 or 9, wherein the predetermined number of perceived state signals received from the user interface correspond to previous perceived state signals that indicate the user's perceived glucose state is higher than a high glucose setpoint.

11. The drug delivery system of one of the preceding embodiments, wherein the user interface is operable to: present a prompt requesting an input indicating the perceived glucose state of the user.

12. A method for adjusting a drug delivery dosage based on a user's perceived glucose state, the method comprising:

receiving a signal from the user interface indicating a perceived glucose state of the user;
evaluating the perceived glucose state of the user indicated by the signal using a glucose measurement value corresponding in time to when the signal was received;
determining an adjustment to a drug delivery algorithm based on the perceived glucose state; and
utilizing the adjustment in a determination of a drug delivery dosage to be administered to a user.

13. The method of embodiment 12, further comprising: based on a result of the perceived glucose state evaluation, assigning a trust index to the user.

14. The method of embodiment 12 or 13, further comprising: selecting a trust increment value based on how closely the perceived glucose state of the user matches the glucose measurement value obtained since the signal was received.

15. The method of one of embodiments 12 to 14, further comprising: comparing the glucose measurement value to various glucose thresholds; based on a result of the comparison, selecting the trust increment value from a plurality of trust increment values.

16. The method of one of embodiments 12 to 15, wherein the selected trust increment value is an indicator of the user's reliability in perceiving their own glucose state.

17. The method of one of embodiments 12 to 16, further comprising: calculating the trust index by increasing a trust level associated with the perceived glucose state based on the selected trust increment value.

18. The method of embodiment 17, wherein the trust level represents a sum of trust increment values selected for a predetermined number of glucose measurement values obtained following receipt of the signal from the user interface.

19. The method of one of embodiments 12 to 18, wherein calculating the trust index further comprises: calculating trust levels for a predetermined number of signals received from the user interface; and determining an average trust level of the trust levels for the predetermined number of signals.

20. The method of one of embodiments 12 to 19, wherein the predetermined number of signals received from the user interface correspond to signals that indicate the user's perceived glucose state is lower than a low glucose setpoint.

21. The method of one of embodiments 12 to 20, wherein the predetermined number of signals received from the user interface correspond to signals that indicate the user's perceived glucose state is higher than a high glucose setpoint.

22. A drug delivery system, comprising:

at least one memory storing programming code operable to control delivery of insulin according to a predetermined insulin dosage;
a user interface operable to accept an input indicating a perceived glucose state of a user; and
at least one processor operable to execute the programming code, wherein the programming code causes the processor to:

receive, at a first time from the user interface, a perceived state signal indicating a perceived state of a user's glucose;
retrieve a glucose measurement value closest in time to the first time;
evaluate how well the glucose measurement value corroborates the perceived state of the user's glucose indicated by the received perceived state signal; and
modify delivery of an insulin dosage based on the evaluation.

23. The drug delivery system of embodiment 22, wherein the programming code further causes the processor to: calculate a trust index based on the evaluation, wherein the trust index is calculated using a function that incorporates a predetermined number of prior glucose evaluations.

24. The drug delivery system of embodiment 22 or 23, wherein the programming code further causes the processor, calculating the trust index, to: select a trust increment value based on how closely the perceived state of the user's glucose corroborates the glucose measurement value.

25. The drug delivery system of one of embodiments 22 to 24, wherein the programming code further causes the processor, when selecting the trust increment value, to: compare the glucose measurement value to various glucose thresholds; based on a result of the comparison, select the trust increment value from a plurality of trust increment values.

26. The drug delivery system of one of embodiments 22 to 25, wherein the selected trust increment value is an indicator of the user's reliability in perceiving their own glucose state.

27. The drug delivery system of one of embodiments 22 to 25, wherein the programming code further causes the processor, when calculating the trust index, to: increase a trust level associated with the perceived state of the user's glucose state based on the selected trust increment value.

28. The drug delivery system of one of embodiments 22 to 27, wherein the trust level represents a sum of trust increment values selected for a predetermined number of glucose measurement values obtained following receipt of the signal.

29. The drug delivery system of one of embodiments 22 to 28, wherein the programming code further causes the processor, when calculating the trust index, to: calculate trust levels for a predetermined number of perceived state signals received from the user interface; and determine an average trust level of the trust levels for the predetermined number of perceived state signals.

30. The drug delivery system of one of embodiments 22 to 29, wherein the predetermined number of perceived state signals received from the user interface correspond to previous perceived state signals that indicate the perceived state of the user's glucose is lower than a low glucose setpoint.

31. The drug delivery system of one of embodiments 22 to 30, wherein the predetermined number of signals received from the user interface correspond to previous perceived state signals that indicate the perceived state of the user's glucose is higher than a high glucose setpoint.

32. The drug delivery system of one of embodiments 1 to 11 or one of embodiments 22 to 31, wherein the system comprises a wearable or on-body drug delivery device and a user device, wherein the wearable or on-body drug delivery device and the user device are designed to communicate wirelessly.

33. The drug delivery system of embodiment 32, wherein the at least one memory and the at least one processor are either included in the drug delivery device or in the user device, or in case of at least two processors and at least two memories, a first processor and a first memory is included in the user device, and a second processor and a second memory is included in the drug delivery device.

34. The drug delivery system of embodiment 33, wherein the user interface comprises at least one first indicator button and at least one second indicator button, wherein the at least one first indicator button is designed to indicate or input at least one perceived low glucose user state, and wherein the at least one second indicator button is designed to indicate or input at least one perceived high glucose user state.

## Claims

1. A drug delivery system, comprising:

   a memory storing programming code operable to control delivery of a drug;
   a user interface operable to accept an input indicating a perceived glucose state of a user; and
   a processor operable to execute the programming code, wherein the programming code causes the processor to:

   receive a perceived state signal from the user interface indicating the perceived glucose state of the user;
   evaluate the perceived glucose state of the user indicated by the perceived state signal using a glucose measurement value corresponding in time to when the perceived state signal was received;
   determine an adjustment to a drug delivery algorithm; and
   utilize the adjustment in a determination of a drug delivery dosage to be administered to a user.

2. The drug delivery system of claim 1, wherein the user interface comprises at least one first indicator button and at least one second indicator button, wherein the at least one first indicator button is designed to indicate or input at least one perceived low glucose user state, and wherein the at least one second indicator button is designed to indicate or input at least one perceived high glucose user state.

3. The drug delivery system of claim 1 or 2, wherein the programming code further causes the processor to:
based on a result of the perceived glucose state evaluation, assign a trust index to the user indicating the user's reliability in assessing the user's own glucose state, in particular a first trust index indicating the reliability in assessing a perceived low glucose state and/or a second trust index indicating the reliability in assessing a perceived high glucose state.

4. The drug delivery system of one of the preceding claims, wherein the programming code further causes the processor, when evaluating the perceived glucose state of the user indicated by the perceived state signal, to:

select a trust increment value based on how closely the perceived glucose state of the user matches the glucose measurement value obtained since the perceived state signal was received.

5. The drug delivery system of claim 4, wherein the programming code further causes the processor, when selecting the trust increment value, to:

compare the glucose measurement value to various glucose thresholds;
based on a result of the comparison, select the trust increment value from a plurality of trust increment values.

6. The drug delivery system of claim 4 or 5, wherein the selected trust increment value is an indicator of the user's reliability in perceiving their own glucose state.

7. The drug delivery system of one of claims 3 to 6, wherein the programming code further causes the processor, to: calculate the trust index by increasing a trust level associated with the perceived glucose state based on the selected trust increment value.

8. The drug delivery system of claim 7, wherein the trust level represents a sum of trust increment values selected for a predetermined number of glucose measurement values obtained following receipt of the perceived state signal.

9. The drug delivery system of one of claims 3 to 8, wherein the programming code further causes the processor, when calculating the trust index, to:

calculate trust levels for a predetermined number of perceived state signals received from the user interface; and
determine an average trust level of the trust levels for the predetermined number of perceived state signals.

10. The drug delivery system of one of the preceding claims, wherein the predetermined number of perceived state signals received from the user interface correspond to previous perceived state signals that indicate the user's perceived glucose state is lower than a low glucose setpoint.

11. The drug delivery system of one of the preceding claims, wherein the predetermined number of perceived state signals received from the user interface correspond to previous perceived state signals that indicate the user's perceived glucose state is higher than a high glucose setpoint.

12. The drug delivery system of one of the preceding claims, wherein the user interface is operable to: present a prompt requesting an input indicating the perceived glucose state of the user.

13. The drug delivery system of one of the preceding claims, wherein the system comprises a wearable or on-body drug delivery device and a user device, wherein the wearable or on-body drug delivery device and the user device are designed to communicate wirelessly.

14. The drug delivery system of claim 13, wherein the at least one memory and the at least one processor are either included in the drug delivery device or in the user device, or in case of at least two processors and at least two memories, a first processor and a first memory is included in the user device, and a second processor and a second memory is included in the drug delivery device.

15. The drug delivery system of claims 13 or 14, wherein the trust index is used to modify the user's target glucose concentration which is used by the processor when executing a control algorithm causing the drug delivery device to deliver doses of the drug or therapeutic agent to the user at predetermined intervals or as needed to bring glucose measurement values to a target glucose value.

FIG. 1

**FIG. 2A**

EP 4 439 575 A1

**FIG. 2B**

300

9:40 AM

Manual

DASHBOARD INSULIN INFO

IOB **0.15 U** **Minimal Risk**

# 121 →

302

304a

I Feel
Low

mg/dL

I Feel
High

304b

● ○ ○

**LAST BOLUS** **CGM GRAPH**

## 6.05
Units

Feb 10 (7:32 pm)

VIEW

**FIG. 3**

EP 4 439 575 A1

```
                                                                          400
  ┌─────────────────────────────────────────────────────────┐
  │ Receive a signal from the user interface indicating a     │
  │ perceived blood glucose state of the user                 │ ─ 402
  └─────────────────────────────────────────────────────────┘
                              │
                              ▼
  ┌─────────────────────────────────────────────────────────┐
  │ Evaluate the perceived blood glucose state of the user    │
  │ indicated by the signal using a blood glucose             │ ─ 404
  │ measurement value corresponding in time to when the       │
  │ signal was received                                       │
  └─────────────────────────────────────────────────────────┘
                              │
                              ▼
  ┌─────────────────────────────────────────────────────────┐
  │ Based on a result of the evaluation, assign a trust index │ ─ 406
  │ to the user                                               │
  └─────────────────────────────────────────────────────────┘
                              │
                              ▼
  ┌─────────────────────────────────────────────────────────┐
  │ Determine an adjustment to a drug delivery algorithm      │ ─ 408
  │ based on the assigned trust index                         │
  └─────────────────────────────────────────────────────────┘
                              │
                              ▼
  ┌─────────────────────────────────────────────────────────┐
  │ Utilize the adjustment in a determination of a drug       │ ─ 410
  │ delivery dosage to be administered to a user              │
  └─────────────────────────────────────────────────────────┘
```

FIG. 4

500

Receive a signal from the user interface indicating a perceived blood glucose state of the user — 502

Receive a new blood glucose measurement and compare the blood glucose measurement value to blood glucose thresholds — 504

Based on a result of the comparison, select a trust increment value from a plurality of trust increment values — 506

Increase the trust level associated with the perceived blood glucose state based on the selected trust increment value — 508

Reached predetermined number of blood glucose measurements? — 510

No

Yes

Complete calculation of the trust level for the perceived blood glucose state of the user — 512

FIG. 5A

FIG. 5B

EP 4 439 575 A1

EP 4 439 575 A1

600

Receive a new signal from the user interface indicating a perceived blood glucose state of the user — 602

Calculate a trust level for the perceived blood glucose state of the user — 604

Reached predetermined number of signals received? — 606

No

Yes

Determine an average trust level of the trust levels for the predetermined number of signals — 608

FIG. 6

Calculate the trust index of the user based on the average trust level — 610

**FIG. 7**

EP 4 439 575 A1

Europäisches
Patentamt

European
Patent Office

Office européen
des brevets

# EUROPEAN SEARCH REPORT

Application Number

EP 24 16 7425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/244880 A1 (LEE JOON BOK [US] ET AL) 12 August 2021 (2021-08-12) * paragraphs [0006], [0007], [0026], [0035], [0044], [0055], [0065], [0074]; figures 2,3 * ----- | 1-15 | INV. G16H20/17 A61M5/172 A61B5/145 A61B5/00 A61M5/142 |
| A | US 11 160 925 B1 (LY TRANG [US] ET AL) 2 November 2021 (2021-11-02) * the whole document * ----- | 1-15 | |

TECHNICAL FIELDS
SEARCHED (IPC)

G16H
A61B
A61M

The present search report has been drawn up for all claims

| Place of search | Date of completion of the search | Examiner |
|---|---|---|
| Munich | 18 July 2024 | Mühlbauer, Max |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons
.....................................................................................................
& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 24 16 7425

This annex lists the patent family members relating to the patent documents cited in the above-mentioned European search report.
The members are as contained in the European Patent Office EDP file on
The European Patent Office is in no way liable for these particulars which are merely given for the purpose of information.

18-07-2024

| Patent document cited in search report | | Publication date | Patent family member(s) | | Publication date |
|---|---|---|---|---|---|
| US 2021244880 | A1 | 12-08-2021 | EP | 4104183 A1 | 21-12-2022 |
| | | | US | 2021244880 A1 | 12-08-2021 |
| | | | WO | 2021163324 A1 | 19-08-2021 |
| US 11160925 | B1 | 02-11-2021 | AU | 2022200293 B1 | 07-07-2022 |
| | | | CA | 3145377 A1 | 24-03-2022 |
| | | | CA | 3164991 A1 | 24-03-2022 |
| | | | EP | 4036934 A1 | 03-08-2022 |
| | | | EP | 4285387 A1 | 06-12-2023 |
| | | | EP | 4285388 A1 | 06-12-2023 |
| | | | US | 11160925 B1 | 02-11-2021 |
| | | | US | 2022240847 A1 | 04-08-2022 |
| | | | US | 2022241504 A1 | 04-08-2022 |
| | | | WO | 2022164737 A1 | 04-08-2022 |
| | | | WO | 2022164738 A1 | 04-08-2022 |

EPO FORM P0459

For more details about this annex : see Official Journal of the European Patent Office, No. 12/82